# DEMANDE DE BREVET EUROPEEN

(11) **EP 1 157 683 A2**
(43) Date de publication de la demande: **28.11.2001**
(21) Numéro de dépôt: 01401041.7
(22) Date de dépôt: 24.04.2001
(51) Int. Cl.: A61K 7/06, A61K 7/48

(54) **Utilisation de fibres comme agent anti-pollution, notamment dans une composition cosmétique**

(30) Priorité: 22.05.2000 FR 0006510
(71) Demandeur: L'OREAL, 75008 Paris (FR)
(72) Inventeur: Afriat, Isabelle, 75003 Paris (FR)
(74) Mandataire: Rasson, Catherine

(57) **Abrégé**

La présente demande se rapporte à l'utilisation de fibres dans une composition cosmétique pour application topique sur la peau, le cuir chevelu et/ou les cheveux, comme agent anti-pollution, ou pour la préparation d'une composition destinée à protéger les matières kératiniques contre les effets nocifs de la pollution.

Les fibres utilisées ont notamment une longueur (L) allant de 1 µm à 10 mm et ont une section comprise dans un cercle de diamètre (D) allant de 1 nm à 100 µm. Comme fibres, on peut utiliser en particulier des fibres de polyamide.

## Description

La présente demande concerne l'utilisation de fibres dans une composition cosmétique comme agent anti-pollution, ou pour la préparation d'une composition destinée à protéger les matières kératiniques contre les effets nocifs de la pollution.

Certains milieux urbains sont régulièrement soumis à des pics de pollution. L'individu dans son environnement quotidien et particulièrement en zone urbaine, peut être soumis à de multiples agressions au niveau des matières kératiniques, et notamment la peau, le cuir chevelu et les cheveux, par différents polluants aériens.

Parmi les polluants pouvant exercer des effets délétères sur les matières kératiniques, les gaz toxiques tels que l'ozone, le monoxyde de carbone, les oxydes d'azote ou les oxydes de soufre, sont l'un des constituants majeurs. Il a été constaté que ces gaz toxiques favorisent la desquamation des matières kératiniques, les rendent sales et ternes. De même ces gaz entraînent une asphyxie cellulaire au niveau desdites matières kératiniques.

On sait par ailleurs que les métaux lourds (plomb, cadmium, mercure) sont des polluants atmosphériques dont les émissions ont notablement augmenté, notamment en milieu urbain ou industriel. Outre certains effets toxiques qui leur sont propres, les métaux lourds ont la propriété de diminuer l'activité des moyens de défense cellulaire contre les radicaux libres [voir par exemple R.S. Dwivedi, J. Toxicol. Cut. & Ocular Toxical. 6(3), 183-191 (1987)]. Ainsi, les métaux lourds aggravent les effets toxiques des polluants gazeux en diminuant l'efficacité des moyens naturels de défense, et provoquent une accélération du phénomène de vieillissement cellulaire. Ceci est vrai en particulier pour les matières kératiniques et notamment la peau, le cuir chevelu et les cheveux qui sont en contact direct et permanent avec le milieu extérieur.

Ainsi, les effets nocifs de la pollution sur les matières kératiniques affectent la respiration cellulaire et se traduisent par un vieillissement accéléré de la peau, avec un teint terne et la formation précoce de rides ou ridules, et aussi par une diminution de la vigueur des cheveux qui prennent aussi un aspect terne. En outre, du fait de la pollution, peau et cheveux se salissent plus rapidement. De plus, la pollution peut provoquer des phénomènes d'allergie sur la peau.

Ainsi, il existe le besoin de compositions permettant d'éviter les effets néfastes dus aux polluants (gaz ou métaux lourds), de façon à protéger les matières kératiniques.

Il a été maintenant constaté de manière tout à fait surprenante que l'utilisation de fibres, et notamment de fibres de polyamide, permettait de protéger les matières kératiniques telles que peau, cuir chevelu et cheveux, des effets des polluants.

Certes, il est connu, par exemple par le document JP07-196440, des compositions cosmétiques contenant des fibres de polyamide courtes, celles-ci donnant aux dites compositions un toucher velouté et une bonne tenue cosmétique. Toutefois, aucun document ne décrit que les fibres puissent avoir des propriétés de protection contre la pollution.

Ainsi, la présente invention a pour objet l'utilisation de fibres dans une composition cosmétique pour application topique sur la peau, le cuir chevelu et/ou les cheveux, comme agent anti-pollution.

On entend ici par « application topique » une application externe sur les matières kératiniques, que sont notamment la peau, le cuir chevelu, les cils, les sourcils, les ongles et les muqueuses.

L'invention a aussi pour objet l'utilisation de fibres pour la préparation d'une composition destinée à protéger les matières kératiniques contre les effets nocifs de la pollution.

Outre un bon effet anti-pollution, les compositions contenant les fibres ont de bonnes propriétés cosmétiques : confort à l'application, texture facile à étaler et douce.

Les fibres utilisables dans la composition de l'invention peuvent être des fibres hydrophiles ou hydrophobes, d'origine synthétique ou naturelle, minérale ou organique.

Ces fibres peuvent être courtes ou longues, unitaires ou organisées par exemple tressées. Leur forme ou morphologie peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres peuvent avoir une longueur (L) allant de 1 µm (0,001 mm) à 10 mm, de préférence de 0,1 µm à 5 mm et mieux de 0,1 mm à 1,5 mm. Leur section peut être comprise dans un cercle de diamètre (D) allant de 1 nm (0,001 µm) à 100 µm, de préférence allant de 1 nm (0,001 µm) à 50 µm et mieux de 5 µm à 40 µm.

De préférence, les fibres utilisées selon la présente invention ont un facteur de forme, c'est-à-dire un rapport L/D (longueur/diamètre) allant de 3,5 à 2500, mieux de 5 à 500 et encore mieux de 5 à 150.

Le titre des fibres est souvent donné en denier ou décitex. Le denier est le poids en gramme pour 9 km de fil. De préférence, les fibres utilisées selon l'invention ont un titre allant de 0,15 à 30 deniers, et mieux de 0,18 à 18 deniers.

Le facteur de forme, le titre et la morphologie des fibres sont les trois facteurs importants pour définir une fibre.

Les fibres peuvent être celles utilisées dans la fabrication des textiles et notamment des fibres de soie, de coton, de laine, de lin, de cellulose extraites notamment du bois, des légumes ou des algues, de polyamide (Nylon® ), de cellulose modifiée (rayonne, viscose, acétate notamment d'acétate de rayonne), de poly-p-phénylène téréphtamide notamment de Kevlar® , en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-dessus, comme des fibres de polyamide/polyester.

On peut aussi utiliser les fibres synthétiques résorbables utilisées en chirurgie, comme les fibres préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson) et les fils d'acier inoxydable (Acier de la société Johnson & Johnson).

On peut aussi utiliser des mélanges des fibres citées ci-dessus.

Par ailleurs, les fibres peuvent être traitées ou non en surface, enrobées ou non. Comme fibres enrobées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou traitement de surface induisant des effets de couleurs/hologrammes (fibre Lurex de la société Sildorex, par exemple).

Selon leurs propriétés, les fibres utilisées selon la présente invention peuvent être introduites dans un milieu aqueux, un milieu huileux ou dans une poudre.

Les fibres utilisables selon l'invention sont de préférence choisies parmi les fibres de polyamide, les fibres de poly-p-phénylènetéréphtamide, les fibres de coton et leurs mélanges. Leur longueur peut aller de 0,1 à 10 mm, de préférence de 0,1 à 1 mm, leur diamètre moyen peut aller de 5 à 50 µm et le facteur de forme va de préférence de 5 à 150.

En particulier, on peut utiliser les fibres de polyamide commercialisées par les Etablissements P. Bonte sous le nom Polyamide 0,9 Dtex 0,3 mm, ayant un diamètre moyen de 15 à 20 µm, un titre d'environ 0,9 dtex (0,81 denier) et une longueur allant de 0,3 mm à 1,5 mm. On peut aussi utiliser les fibres de poly-p-phénylène téréphtamide de diamètre moyen de 12 µm et de longueur d'environ 1,5 mm comme celles vendues sous le nom de Kevlar Floc par la société Du Pont Fibres. Ces fibres de polyamide sont de préférence introduites dans un milieu huileux ou par voie sèche dans une poudre.

On peut aussi utiliser des fibres de coton ayant un diamètre moyen de 20 µm, une longueur allant de 0,3 mm, et un facteur de forme de 15.

Les fibres peuvent être présentes dans la composition selon l'invention en une quantité allant par exemple de 0,1 à 20 % en poids, de préférence de 0,5 à 15 % en poids et mieux de 2 à 15 % en poids par rapport au poids total de la composition.

La composition peut aussi contenir d'autres actifs anti-pollution tels que par exemple les sphingolipides décrits dans le document EP-A-0 577 718.

Les compositions à application topique, et notamment cosmétiques, utilisées selon l'invention contiennent un milieu physiologiquement acceptable, c'est-à-dire un milieu compatible avec la peau, les lèvres, le cuir chevelu, les cils, les yeux, les ongles et/ou les cheveux. Ce milieu physiologiquement acceptable peut être plus particulièrement constitué d'eau et éventuellement d'un solvant organique physiologiquement acceptable choisi par exemple parmi les alcools inférieurs comportant de 1 à 4 atomes de carbone comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol. Ce peut être aussi un milieu anhydre, notamment un milieu huileux contenant des huiles et/ou matières grasses autres que les huiles.

Le milieu physiologiquement acceptable a un pH compatible avec la peau, allant de préférence de 3 à 8 et mieux de 4,5 à 7.

Quand la composition comporte un milieu aqueux ou hydroalcoolique, il est possible d'ajouter une phase grasse (ou huileuse) dans ce milieu, afin que les compositions de l'invention soient plus douces et plus nourrissantes.

Ainsi, les compositions selon l'invention contenant les agents anti-pollution tels que définis ci-dessus peuvent se présenter sous toutes les formes galéniques classiquement utilisées pour une application topique et notamment sous forme de solutions aqueuses, hydroalcooliques ou huileuses, d'émulsions huile-dans-eau (H/E) ou eau-dans-huile (E/H) ou multiple, de gels aqueux ou huileux, de produits anhydres liquides, pâteux ou solides, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique. Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Comme huiles utilisables dans les compositions de l'invention, on peut citer les huiles minérales telles que l'huile de vaseline ; les huiles d'origine végétale telles que la fraction liquide du beurre de karité, l'huile de tournesol ; les huiles d'origine animale telles que le perhydrosqualène ; les huiles de synthèse telles que le polyisobutène hydrogénée ; les huiles de silicone non volatiles ou volatiles telles que les cyclométhicones comme la cyclopentasiloxane ; et les huiles fluorées telles que les perfluoropolyéthers. On peut aussi utiliser, comme matières grasses autres que les huiles, des alcools gras, des acides gras, des cires. La phase huileuse de l'émulsion peut contenir aussi des gommes telles que les gommes de silicone, des résines et notamment les résines de silicone, et les élastomères de silicone tels que les produits commercialisés sous la dénomination "KSG" par la société Shin-Etsu, sous la dénomination "Trefil" par la société Dow Corning ou sous la dénomination "Gransil" par la société General Electric.

Selon un mode particulier de réalisation de l'invention, la composition contenant les fibres est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90^{R} par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

De façon connue, la composition cosmétique ou dermatologique de l'invention peut contenir également des adjuvants habituels dans le domaine cosmétique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur, les matières colorantes, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme actifs, on peut ajouter aux fibres utilisées dans la composition selon l'invention, d'autres agents connus pour favoriser la lutte contre la pollution. Comme autres agents anti-pollution, on peut citer par exemple les métallothionéines décrites dans le document EP-A-557 042, les sphingolipides décrits dans le document EP-A-577 718, et tout autre composé ayant la propriété d'éviter les effets néfastes des polluants.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Les compositions utilisées selon l'invention peuvent notamment constituer un produit de soin et/ou de maquillage. Elles peuvent être utilisées notamment pour protéger l'organisme, en particulier les matières kératiniques, contre les effets de la pollution, notamment pour améliorer la respiration cellulaire et/ou diminuer la desquamation et/ou pour éviter de ternir ou de salir les matières kératiniques, et notamment la peau.

Ainsi, un autre objet de l'invention consiste en un procédé de traitement cosmétique en vue de protéger les matières kératiniques contre les effets de la pollution, consistant à appliquer sur les matières kératiniques une quantité efficace d'une composition cosmétique contenant des fibres.

L'invention a aussi pour objet un procédé de traitement cosmétique des matières kératiniques en vue d'améliorer leur respiration cellulaire et/ou de diminuer leur desquamation et/ou d'éviter de les ternir et/ou de les salir, consistant à appliquer sur les matières kératiniques une quantité efficace d'une composition cosmétique contenant des fibres.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook) et les quantités en pourcentage en poids sauf mention contraire.

### Exemple 1 : Emulsion E/H

### Phase A :

- Cire microcristalline 1,2 %
- Polyisobutène hydrogéné 4,6 %
- Propylparaben (conservateur) 0,01 %
- Polyaminopropyl biguanide (conservateur) 1 %
- Sulfate de magnésium 0,7 %
- Silice 0,5 %
- Polymethylsesquioxane 0,4 %
- Copolymère éthylène/acide acrylique 0,6 %
- Acrylates copolymer 0,04 %

### Phase B :

- Cyclomethicone/Disteardimonuim Hectorite/ alcool
   (Mélange 85/10/5 vendu sous le nom Bentone Gel VS-5V par Elementis Specialties) 2 %
- Fibres de polyamide
   (Polyamide 0,9 Dtex, 0,3 mm - Société Paul Bonte) 10 %
- Cyclopentasiloxane 9 %
- Dimethicone/vinyl dimethicone crosspolymer/dimethicone
   (KSG 16 vendu par Shin-Etsu) 2 %

### Phase C :

- Dimethicone/vinyl dimethicone crosspolymer/dimethicone
   (KSG 21 vendu par Shin-Etsu) 2 %
- Cyclopentasiloxane 9 %

### Phase D :

- Glycérine 5 %
- Eau qsp 100 %

### Mode opératoire :

On prépare séparément la phase A et la phase B, on les mélange et on passe le mélange à la tri-cylindre plusieurs fois. On ajoute le ménage obtenu à la phase C et on homogénéise le tout sous agitation. Puis on fait l'émulsion sous agitation en versant petit à petit la phase aqueuse D dans le mélange des phases A, B et C.

### Exemple 2 : Emulsion H/E

### Phase A :

### 1/prémélange A'

- Cire microcristalline 1,2 %
- Polyisobutène hydrogéné 4,6 %
- Silice 0,5 %
- Copolymère éthylène/acide acrylique 0,6 %
- Acrylates copolymer 0,04 %
- Polymethylsesquioxane 0,4 %
- Dimethicone/vinyl dimethicone crosspolymer and dimethicone (KSG 6 de la société Shin-Etsu) 2,57 %
- Conservateurs 0.015 %

### 2/ prémélange A"

- Fibres de polyamide
   (Polyamide 0,9 Dtex, 0,3 mm - Société Paul Bonte) 10 %
- Cyclomethicone/Disteardimonuim Hectorite/ alcool
   (Mélange 85/10/5 vendu sous le nom Bentone Gel VS-5V par Elementis Specialties) 2 %
- Cyclopentasiloxane 28 %
- Dimethicone/vinyl dimethicone crosspolymer and dimethicone (KSG 16 de la société Shin-Etsu) 2 %

### Phase B :

- Conservateurs 1 %
- Carbomer 0,5 %
- Acrylates/C10-30 alkyl acrylate crosspolymer
   (Pemulen de la société Goodrich) 0,5 %
- Eau qsp 100 %

### Phase C :

- Triéthanolamine 0,6 %
- Eau 2 %

### Mode opératoire :

On réalise tout d'abord le mélange des prémélanges A' et A" de la phase A en incorporant le prémélange A" dans le prémélange A' et en passant le mélange à la tri-cylindre plusieurs fois. On prépare parallèlement la phase B en dispersant le Pemulen et le carbomer dans l'eau additionnée des conservateurs sous forte agitation (type moritz). On réalise l'émulsion en versant petit à petit la phase A dans la phase B sous agitation. Puis on neutralise en ajoutant la phase C.

### Exemple 3 : Emulsion H/E

### Phase A :

- Fibres de polyamide
   (Polyamide 0,9 Dtex, 0,3 mm - Société Paul Bonte) 12 %
- Cyclomethicone/Disteardimonuim Hectorite/ alcool (Mélange 85/10/5 vendu sous le nom Bentone Gel VS-5V
   par Elementis Specialties) 2 %
- Cyclopentasiloxane 12 %
- Dimethicone/vinyl dimethicone crosspolymer and dimethicone (KSG 16 de la société Shin-Etsu) 4 %

### Phase B :

- Conservateurs 1 %
- Carbomer 0,3 %
- Acrylates/C10-30 alkyl acrylate crosspolymer
   (Pemulen de la société Goodrich) 0,3 %
- Eau qsp 100 %

### Phase C :

- Triéthanolamine 0,6 %
- Eau 2 %

### Mode opératoire :

On mélange les constituants de la phase A et on passe ce mélange à la tricylindre plusieurs fois. Parallèlement, on prépare la phase B en dispersant sous forte agitation (type moritz) le Pemulen et le carbomer dans l'eau additionnée des conservateurs. On réalise l'émulsion en versant petit à petit la phase A dans la phase B sous agitation. On neutralise ensuite en ajoutant la phase C.

### Test pour la mise en évidence de l'effet protecteur des fibres

### Efficacité anti-pollution sur peau reconstruite

Les émulsions des exemples 1 et 2 (2 mg/cm²) ont été appliquées à la surface d'épidermes de peau reconstruite et laissées à leur contact pendant 30 minutes à température ambiante. Les épidermes de peau reconstruites utilisés sont vendus par la société EPISKIN (LYON, France) et. les milieux de culture sont ceux inclus dans le kit vendu par le fournisseur. Les épidermes ont été utilisés au stade de différentiation J13.

Après les 30 minutes de contact, des particules radio-marquées au carbone 14 ont été déposées sur les épidermes et laissées à leur contact pendant 2 heures dans le milieu de maintenance habituel des épidermes. Puis les épidermes ont été retirés de leur milieu de maintenance et lavés plusieurs fois au tampon PBS (= phosphate Buffer Saline). Les lavages permettent de débarrasser les épidermes des particules faiblement adsorbées sans éliminer l'émulsion initialement appliquée. Les taux de particules radio-marquées résiduels ont été ensuite évalués par la mesure de la radioactivité du carbone 14 additionné aux particules. Le tableau ci-dessous donne les résultats en pourcentage de particules résiduelles par rapport à la quantité de particules déposées :

**Tableau**

| | % par rapport à la quantité de particules déposées |
|---|---|
| Zone non traitée | 36,2 % |
| Zone traitée avec émulsion de l'exemple 1 | 14,3 % |
| Zone traitée avec émulsion de l'exemple 2 | 26,9 % |

Ces résultats montrent que les émulsions selon l'invention permettent une bonne protection de la peau contre les particules polluantes et que l'émulsion eau-dans-huile de l'exemple 1 a encore une meilleure protection que l'émulsion huile-dans-eau de l'exemple 2.

## Revendications

1. Utilisation de fibres dans une composition cosmétique pour application topique sur la peau, le cuir chevelu et/ou les cheveux, comme agent anti-pollution.

2. Utilisation de fibres pour la préparation d'une composition destinée à protéger les matières kératiniques contre les effets nocifs de la pollution.

3. Utilisation selon la revendication 1 ou 2, **caractérisée en ce que** les fibres ont une longueur (L) allant de 1 µm à 10 mm.

4. Utilisation selon l'une quelconque des revendications précédentes, **caractérisée en ce que** les fibres ont une section comprise dans un cercle de diamètre (D) allant de 1 nm à 100 µm.

5. Utilisation selon l'une quelconque des revendications précédentes, **caractérisée en ce que** les fibres ont un facteur de forme (L/D) allant de 5 à 150.

6. Utilisation selon l'une quelconque des revendications précédentes, **caractérisée en ce que** les fibres ont un titre allant de 0,15 à 30 deniers.

7. Utilisation selon l'une quelconque des revendications précédentes, **caractérisée en ce que** les fibres sont choisies parmi les fibres de soie, de coton, de laine, de lin, de cellulose extraites notamment du bois, des légumes ou des algues, de polyamide (Nylon® ), de cellulose modifiée, de poly-p-phénylène téréphtamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges.

8. Utilisation selon l'une quelconque des revendications précédentes, **caractérisée en ce que** les fibres sont enrobées.

9. Utilisation selon l'une quelconque des revendications précédentes, **caractérisée en ce que** les fibres sont choisies parmi les fibres de polyamide, les fibres de poly-p-phénylènetéréphtamide, les fibres de coton et leurs mélanges.

10. Utilisation selon l'une quelconque des revendications précédentes, **caractérisée en ce que** les fibres sont présentes en une quantité allant de 0,1 à 20 % en poids par rapport au poids total de la composition.

11. Utilisation selon l'une quelconque des revendications précédentes, **caractérisée en ce que** la composition contient en outre un milieu physiologiquement acceptable.

12. Utilisation selon l'une quelconque des revendications précédentes, **caractérisée en ce que** la composition contient en outre un autre agent anti-pollution.

13. Utilisation selon l'une quelconque des revendications précédentes, **caractérisée en ce que** la composition est sous forme d'une émulsion.

14. Utilisation selon l'une quelconque des revendications précédentes, **caractérisée en ce que** la composition constitue un produit de soin et/ou de maquillage.

15. Procédé de traitement cosmétique en vue de protéger les matières kératiniques contre les effets de la pollution, consistant à appliquer sur les matières kératiniques une quantité efficace d'une composition cosmétique contenant des fibres.

16. Procédé de traitement cosmétique des matières kératiniques en vue d'améliorer leur respiration cellulaire et/ou de diminuer leur desquamation et/ou d'éviter de les ternir et/ou de les salir, consistant à appliquer sur les matières kératiniques une quantité efficace d'une composition cosmétique contenant des fibres.

17. Procédé selon la revendication 15 ou 16, **caractérisée en ce que** la matière kératinique est la peau.
